# EUROPÄISCHE PATENTSCHRIFT

(11) **EP 0 978 510 B1**
(45) Veröffentlichungstag und Bekanntmachung des Hinweises auf die Patenterteilung: **22.04.2009**
(21) Anmeldenummer: 99114036.9
(22) Anmeldetag: 20.07.1999
(51) Int. Cl.: C07D 251/26

(54) **Verfahren zur Herstellung von Alkalimetall-oder Erdalkalimetallsalzen von 2,4-Dichlor-6-hydroxy-s-triazin**
Process for the preparation of alkali metal or alkali earth metal salts of 2,4-dichlor-6-hydroxy-s-triazin
Procédé pour la préparation de sels de métal alcalin ou alcalino-terreux de la 2,4-dichlor-6-hydroxy-s-triazine

(30) Priorität: 07.08.1998 DE 19835774
(43) Veröffentlichungstag der Anmeldung: 09.02.2000
(73) Patentinhaber: Evonik Degussa GmbH, 45128 Essen (DE)
(72) Erfinder: Schmidt, Manfred, Dr., 63571 Gelnhausen (DE); Klatte, Christoph, 63580 Gründau (DE); Kunz, Kurt, 63633 Birstein (DE); Leutner, Josef, 63920 Grossheubach (DE); Ohlemacher, Jürgen, 63477 Maintal (DE); Krimmer, Hans-Peter, Dr., 63128 Dietzenbach (DE)

(56) Entgegenhaltungen:
- EP-A- 0 597 312
- DD-A- 115 121
- DE-A- 2 910 726
- DE-A- 4 424 733

## Beschreibung

Die Erfindung betrifft ein Verfahren zur Herstellung von Alkalimetall- oder Erdalkalimetallsalzen von 2,4-Dichlor-6-hydroxy-s-triazin, insbesondere des Na-Salzes (= DCHT-Na), durch Umsetzung von Cyanurchlorid mit einem Alkali- oder Erdalkalihydroxid. Das Verfahren läßt sich auch im technischen Maßstab sicher betreiben.

Alkalimetallsalze von 2,4-Dichlor-6-hydroxy-s-triazin finden Anwendung als Rohstoff für Reaktivfarbstoffe, als Vernetzer für Photogelatine und neuerdings als formaldehydfreier Vernetzer zur Erzeugung von Knitterfest- und Pflegeleichteigenschaften von Cellulose enthaltenden Fasermaterialien - siehe EP-A 0 616 071. Wegen des zersetzlichen Charakters des festen Triazinderivats wird dieses üblicherweise nicht isoliert, sondern in wäßriger Phase weiterverarbeitet.

In der EP 597 312 wird die Verwendung von Triazinen und u. a. 2,4-Dichlor-6-hydroxy-s-Triazin in der Herstellung polymerer Filme genannt. Die DE 29 10 726 beschreibt lagerstabile wässrige Härtungslösungen für proteinhaltige Materialien, welche ein wasserlösliches Salz des 2,4-Dichlor-6-hydroxy-1,3,5-triazin enthalten. Derartige Derivate werden in der DE 44 24 733 zur Herstellung von faserreaktiven Farbstoffen vorgestellt.

Es ist bekannt, daß Cyanurchlorid mit Alkalihydroxiden zu 2,4-Dichlor-6-hydroxy-s-triazin und 2-Chlor-4,6-dihydroxy-s-triazin hydrolysiert werden kann - siehe Smolin und Rappoport, The Chemistry of Heterocyclyc Compounds, S-Triazines and Derivatives (1967), 53-54. Nach diesem Dokument bleibt die Hydrolyse nicht auf der gewünschten ersten Stufe stehen, sondern das zweite und dritte Chloratom werden zumindest teilweise hydrolysiert.

Gemäß DD-Patent 115 121 wird 2,4-Dichlor-6-hydroxy-s-triazin-Na-Salz, nachfolgend als DCHT-Na abgekürzt, hergestellt durch Zugabe von in Benzol oder Chloroform gelöstem Cyanurchlorid zu einer wäßrigen Lösung von Soda oder Natriumbicarbonat bei 15 °C. Nachteilig sind der Einsatz organischer Lösungsmittel und die CO₂-Entwicklung

Einsatz organischer Lösungsmittel und die CO₂-Entwicklung aus Na₂CO₃ oder NaHCO₃. Im Verfahren gemäß JP-A 59-106474 werden 2,0 bis 2,6 Mol NaHCO₃ pro Mol Cyanurchlorid zusammen mit einem Puffer, etwa einem Phosphatpuffer, zur Hydrolyse eingesetzt.

Wenn Cyanurchlorid mittels Natronlauge anstelle mittels Natriumbicarbonat in DCHT-Na überführt werden soll, wird gemäß SU-A 1051082 wiederum ein Puffer eingesetzt und die Umsetzung bei pH 8,5 bis 8,8 durchgeführt. Der Puffer ist aber, da die Lösung weiter verarbeitet wird, vielfach unerwünscht. Zudem besteht bei dieser Arbeitsweise wegen der begrenzten Pufferkapazität das Risiko eines Absinkens des pH-Werts auf Werte unter 7 und damit zu einer vermehrten Bildung des Di- und Trihydroxytriazinderivats.

K. Matsui und J. Sakomoto zeigen in Yuki Gosei Kagaku 18 (1960), H.3, 175-183 (45-53), daß zur Herstellung von 2,4-Dichlor-6-hydroxy-s-triazin (= DCHT-Na) Natronlauge bei 0 bis 5 °C zu einer Suspension von Cyanurchlorid gegeben werden soll. Zur Herstellung von 2-Chlor-4,6-dihydroxytriazin-Na-Salz (= CDHT-Na) wird dagegen eine Cyanurchloridsuspension in Natronlauge eingetragen. Bei der Nacharbeitung des in diesem Dokument beschriebenen Verfahrens wurde gefunden, daß auf diese Weise zwar im Labormaßstab DCHT-Na hergestellt werden kann, daß dieses Verfahren aber im technischen Maßstab sicherheitstechnische Probleme aufwirft - kaum beherrschbare Freisetzung der Reaktionswärme oder zu geringe Reaktionsgeschwindigkeit.

Aufgabe der vorliegenden Erfindung ist demgemäß, die Hydrolyse von Cyanurchlorid zu DCHT-Na unter Verwendung von Natronlauge so zu verbessern, daß die Umsetzung auch im technischen Maßstab sicher beherrschbar bleibt.

Die Aufgabe wird gelöst durch ein Verfahren zur Herstellung eines Alkalimetall- oder Erdalkalimetallsalzes von 2,4-Dichlor-6-hydroxy-s-triazin, umfassend Hydrolyse von Cyanurchlorid mit wäßrigem Alkali- oder Erdalkalihydroxid bei einer Temperatur von gleich oder unterhalb 10 °C, das dadurch gekennzeichnet ist, daß man 1 bis 2,5 Äquivalente Alkali- oder Erdalkalihydroxid pro Mol Cyanurchlorid in Form einer wäßrigen Lösung oder Suspension vorlegt und hierzu unter Aufrechterhaltung eines pH-Werts im Bereich von 9,5 bis 14 und Kühlung Cyanurchlorid in Pulverform oder in Form einer wäßrigen Suspension zudosiert, im Falle einer unterstöchiometrischen Vorlage an Alkali- oder Erdalkalihydroxid die restliche Menge einschließlich eines Überschusses bis zu 25 % während und/oder nach der Zugabe von Cyanurchlorid zwecks Aufrechterhaltung des pH-Wertes zudosiert und die Umsetzung beendet, wenn sich der pH-Wert bei der gewählten Temperatur im wesentlichen nicht mehr ändert.

Völlig überraschend wurde gefunden, daß bei der erfindungsgemäßen Arbeitsweise trotz des vorlagebedingten Überschusses an Alkali- oder Erdalkalihydroxid Cyanurchlorid mit hoher Selektivität zum entsprechenden DCHT-Salz hydrolysiert werden kann und das 2-Chlor-4,6-dihydroxy-s-triazin-Salz nur in geringer Menge gebildet wird. Das Verfahren läßt sich auch im technischen Maßstab sicher betreiben, weil die Reaktionswärme zuverlässig abgeführt und der pH-Wert oberhalb vorzugsweise 10 gehalten werden kann. An einen zumindest teilweise durchgeführten erfindungsgemäßen Batchansatz kann sich auch eine kontinuierliche Fahrweise anschließen: Hierbei werden in das Reaktionsgemisch des Batchansatzes gleichzeitig und unter Kühlung und pH-Kontrolle eine Cyanurchloridsuspension und eine Alkalilauge oder Erdalkalihydroxidsuspension im etwa stöchiometrischen Verhältnis eindosiert, eine der Zugabemenge entsprechende Menge aus dem Reaktor, dieser ist zweckmäßigerweise als Umlaufreaktor ausgebildet, abgezogen und letztere in einem nachgeschaltenen Reaktor, etwa einem Strömungsrohr, bis zur Beendigung der Umsetzung auf Reaktionstemperatur gehalten.

Bei den erfindungsgemäß herzustellenden Alkalimetallsalzen handelt es sich insbesondere um das Li-, Na- oder K-Salz, bei den Erdalkalimetallsalzen insbesondere um das Mg-, Ca-, Sr- oder Ba-Salz; besonders bevorzugt handelt es sich um das Na-Salz. Die Konzentration der Alkalihydroxidlösung oder Erdalkalihydroxidsuspension wird danach ausgerichtet, welche Endkonzentration an DCHT-Na gewünscht wird, und ob die Kühlung durch direkte Kühlung mittels Eis oder indirekte Kühlung erfolgt. Zum Zwecke einer rascheren Umsetzung kann der vorgelegten Alkalilauge oder Erdalkalihydroxidsuspension oder der Cyanurchloridsuspension ein Netzmittel zugesetzt werden. Eine raschere Cyanurchloridzugabe macht aber auch eine verbesserte Wärmeabfuhr erforderlich.

Obgleich das theoretische Molverhältnis von Alkalihydroxid zu Cyanurchlorid 2 zu 1 ist, kann es zweckmäßig sein, Alkalihydroxid im Überschuß von bis zu 25 %, vorzugsweise bis 10 %, während der letzen Phase der Umsetzung, also nach beendeter Cyanurchloridzugabe, zur Aufrechterhaltung des pH-Werts und Stabilisierung des Reaktionsgemischs zuzugeben. Gemäß einer bevorzugten Ausführungsform wird Alkalihydroxid in einer Menge von 1,2 bis 2,2 Mol, insbesondere 1,5 bis 2,0 Mol, pro Mol Cyanurchlorid vorgelegt. Bei Verwendung von Erdalkalihydroxiden entspricht das zuvor genannte Molverhältnis dem Äquivalentverhältnis.

Von Wichtigkeit ist, den pH-Wert während der Zugabe von Cyanurchlorid und während der Nachreaktion auf Werten über 9,5 , vorzugsweise über 10 zu halten. Der End-pH-Wert sollte bevorzugt im Bereich von 10 bis 12 liegen. Bei einem pH-Wert unter 9,5 besteht die Gefahr eines weiteren Absinkens in den sauren Bereich und damit vermehrte Bildung von Salzen des Dihydroxy-monochlortriazins (= CDHT) und Cyanursäure.

Üblicherweise wird die Umsetzung bei einer Temperatur im Bereich von -10 bis +10 °C, vorzugsweise bei -5 bis +5 °C und insbesondere bei 0 bis 3 °C durchgeführt.

Das Verfahren ist sicher handhabbar und mit hoher Raum-Zeit-Ausbeute betreibbar. Das erhaltene DCHT-Salz enthält das unerwünschte Nebenprodukt CDHT-Salz nur in sehr geringer Menge.

### Beispiel 1

In einem temperierbaren 100-1-Behälter mit Emaillierung wurden 20 kg Cyanurchlorid in ca. 47 kg Wasser suspendiert und auf ca. 0 °C gekühlt. In einem 500-l-Gefäß mit Rührer wurden 50 l Wasser, 60 kg Eis und 15 kg 50 %ige NaOH vorgelegt. Die Temperatur der Mischung sank auf etwa -8 °C.

In Intervallen wurde aus dem 100-l-Behälter CyanurchloridSuspension in die Vorlage gepumpt. Die Temperatur stieg langsam an und wurde durch portionsweise Zugabe von Eis (insgesamt 120 kg für die gesamte Reaktionszeit) bei max. +2 °C gehalten. Gegen Ende der Dosierung begann der pH-Wert zu sinken - er wurde durch Zugabe von 2 kg 50 gew.-%iger NaOH-Lösung zwischen 10 und 11 gehalten. Die Reaktion war, wie sich durch Kontrolle mittels HPLC-Analyse (Säule: 2x Lichrospher 100 RP 18 (5 µm) von Merck) ergab, beendet, als sich der pH bei konstanter Temperatur nicht mehr veränderte. Die Dosierung der Suspension dauerte eine % Stunde, die gesamte Reaktion benötigte etwa 3 Stunden. Die HPLC-Analyse des Reaktionsgemischs nach beendeter Umsetzung zeigte, daß Cyanurchlorid zu 99,7 % in 2,4-Dichlor-6-hydroxy-s-triazin-Na-Salz (= DCHT-Na-Salz), zu 0,2 % in 2-Chlor-4,6-dihydroxy-s-triazin-Na-Salz (= CDHT-Na-Salz) und zu 0,1 % in Na-Salz der Cyanursäure überführt wurde.

### Beispiel 2

20 kg Cyanurchlorid wurden mit insgesamt 16,4 kg 50 gew.-%iger Natronlauge umgesetzt: Vorgelegt wurde ein Gemisch aus 50 kg Wasser, 10 kg Natronlauge (50 Gew.-%) und 60 kg Eis, wobei die Temperatur des Gemischs -5 °C betrug. Unter intensivem Rühren und Kühlen durch Zugabe von Eis (insgesamt ca. 120 kg) wurde innerhalb 45 Minuten eine zuvor auf 0 °C gekühlte wäßrige Suspension aus 20 kg Cyanurchlorid und 50 kg Wasser zudosiert und, soweit erforderlich, der pH-Wert durch Zugabe von Natronlauge auf Werten im Bereich von 10 bis 14 gehalten. Die Temperatur wurde während der Cyanurchloridzugabe (ca. 1 h) und während der sich anschließenden Zugabe der restlichen Menge Natronlauge zwecks Aufrechterhaltung eines pH-Wertes von 10 bis 14 bei 0 bis 1 °C gehalten. Nach 3,5 Stunden war die Umsetzung beendet, der pH-Wert betrug 10,5.

HPLC-Analyse (Fläche-%): 99,4 % DCHT-Na-Salz, 0,5 % CDHT-Na-Salz und 0,1 % Cyanursäure-Na-Salz.

### Beispiel 3

Beispiel 2 wurde wiederholt, wobei jedoch der pH-Wert kurzfristig auf unter 10 abfiel und die Temperatur nach 1,5 Stunden von 0 bis 1 °C auf 3 °C erhöht wurde. Ende der Umsetzung nach 2,5 Stunden. HPLC-Analyse: 96,6 % DCHT-Na-Salz, 3,2 % CDHT-Na-Salz, 0,1 % Cyanursäure-Na-Salz.

## Patentansprüche

1. Verfahren zur Herstellung eines Alkalimetall- oder Erdalkalimetallsalzes von 2,4-Dichlor-6-hydroxy-s-triazin, umfassend Hydrolyse von Cyanurchlorid mit wäßrigem Alkali- oder Erdalkalihydroxid bei einer Temperatur von gleich oder unterhalb 10 °C,
**dadurch gekennzeichnet,**
**daß** man 1 bis 2,5 Äquivalente Alkali- oder Erdalkalihydroxid pro Mol Cyanurchlorid in Form einer wäßrigen Lösung oder Suspension vorlegt und hierzu unter Aufrechterhaltung eines pH-Werts im Bereich von 9,5 bis 14 und Kühlung Cyanurchlorid in Form einer wäßrigen Suspension zudosiert, im Falle einer , unterstöchiometrischen Vorlage an Alkali- oder Erdalkalihydroxid die restliche Menge einschließlich eines Überschusses bis zu 25 % während und/oder nach der Zugabe von Cyanurchlorid zwecks Aufrechterhaltung des pH-Wertes zudosiert und die Umsetzung beendet, wenn sich der pH-Wert bei der gewählten Temperatur im wesentlichen nicht mehr ändert.

2. Verfahren nach Anspruch 1,
**dadurch gekennzeichnet,**
**daß** man unter Einsatz von Natronlauge 2,4-Dichlor-6-hydroxy-s-trazin-Na-Salz herstellt.

3. Verfahren nach Anspruch 2,
**dadurch gekennzeichnet,**
**daß** man 1,2 bis 2,2 Mol, insbesondere 1,5 bis 2,0 Mol Natronlauge pro Mol Cyanurchlorid vorlegt.

4. Verfahren nach einem der Ansprüche 1 bis 3,
**dadurch gekennzeichnet,**
**daß** man die Umsetzung unter Aufrechterhaltung einer Temperatur im Bereich von -5 bis +5 °C, insbesondere 0 bis 3 °C und Aufrechterhaltung eines pH-Wertes von mindestens 10 durchführt.

## Claims

1. A method of producing an alkali metal or alkaline earth metal salt of 2,4-dichloro-6-hydroxy-s-triazine comprising hydrolysing cyanuric chloride with aqueous alkali metal or alkaline earth metal hydroxide at a temperature of equal to or below 10°C, **characterized in that** 1 to 2.5 equivalents of alkali metal or alkaline earth metal hydroxide per mole of cyanuric chloride is initially charged in the form of an aqueous solution or suspension and cyanuric chloride is added with cooling in the form of an aqueous suspension while the pH is maintained at the range from 9.5 to 14, in the case of a substoichiometric initial charge of alkali metal or alkaline earth metal hydroxide the remainder including an excess up to 25% is added during and/or after the addition of cyanuric chloride for the purpose of maintaining the pH and the reaction is terminated when the pH substantially no longer changes at the temperature chosen.

2. A method according to claim 1, **characterized in that** the sodium salt of 2,4-dichloro-6-hydroxy-s-triazine is produced by using aqueous sodium hydroxide solution.

3. A method according to claim 2, **characterized in that** the amount of aqueous sodium hydroxide solution initially charged per mole of cyanuric chloride is in the range from 1.2 to 2.2 mol, in particular in the range from 1.5 to 2.0 mol.

4. A method according to any one of claims 1 to 3, **characterized in that** the reaction is carried out while maintaining a temperature in the range from -5 to +5°C, in particular 0 to 3°C, and maintaining a pH of at least 10.

## Revendications

1. Procédé pour la préparation d'un sel de métal alcalin ou de métal alcalino-terreux de 2,4-dichloro-6-hydroxytriazine-s, comprenant l'hydrolyse de chlorure de cyanuryle par un hydroxyde aqueux de métal alcalin ou de métal alcalino-terreux, à une température égale ou inférieure à 10 °C, **caractérisé en ce qu'**on dispose au préalable 1 à 2,5 équivalents d'hydroxyde de métal alcalin ou de métal alcalino-terreux sous forme de solution ou suspension aqueuse et on y ajoute par addition dosée du chlorure de cyanuryle sous forme d'une suspension aqueuse, en maintenant un pH dans l'intervalle de 9,5 à 14 et en refroidissant, dans le cas d'une disposition au préalable d'hydroxyde de métal alcalin ou de métal alcalino-terreux en quantité inférieure à la quantité stoechiométrique on ajoute par addition dosée la quantité restante, y compris un excès de jusqu'à 25 %, pendant et/ou après l'addition du chlorure de cyanuryle, afin de maintenir le pH, et la réaction est achevée lorsque le pH ne varie pratiquement plus à la température choisie.

2. Procédé selon la revendication 1, **caractérisé en ce qu'**on prépare le sel de Na de 2,4-dichloro-6-hydroxytriazine-s en utilisant une solution d'hydroxyde de sodium.

3. Procédé selon la revendication 2, **caractérisé en ce qu'**on dispose au préalable 1,2 à 2,2 moles, en particulier 1,5 à 2,0 moles de solution d'hydroxyde de sodium par mole de chlorure de cyanuryle.

4. Procédé selon l'une quelconque des revendications 1 à 3, **caractérisé en ce qu'**on effectue la réaction en maintenant une température dans la plage de -5 à +5 °C, en particulier de 0 à 3 °C et en maintenant un pH d'au moins 10.
